Europäisches Patentamt

European Patent Office

Office européen des brevets

(19)

(11) Veröffentlichungsnummer: **0 332 960**
**A1**

## (12) EUROPÄISCHE PATENTANMELDUNG

(21) Anmeldenummer: 89103851.5

(22) Anmeldetag: 04.03.89

(51) Int. Cl.⁴: **A61F 5/04**

(30) Priorität: 12.03.88 DE 8803344 U

(43) Veröffentlichungstag der Anmeldung:
20.09.89 Patentblatt 89/38

(84) Benannte Vertragsstaaten:
**DE FR GB**

(71) Anmelder: **Maier, Roman**
**Zelterweg 6**
**D-6907 Nussloch(DE)**

(72) Erfinder: **Maier, Roman**
**Zelterweg 6**
**D-6907 Nussloch(DE)**

(74) Vertreter: **Geitz, Heinrich, Dr.-Ing.**
**Postfach 2708 Kaiserstrasse 156**
**D-7500 Karlsruhe 1(DE)**

(54) Vorrichtung zum Schienen von Extremitäten, insbesondere von Unterschenkelfrakturen.

(57) Die zum Schienen von Extremitäten, insbesondere von Unterschenkelfrakturen, bestimmte Vorrichtung besitzt ein langgestrecktes Unterteil (11) mit einer einseitig offenen, anatomisch geformten Längsausnehmung (12) und zwei sich an den Längskanten der Längsausnehmung (12) entlang erstreckende sowie letztere zu einem U-förmigen Kanal (13) ergänzende Seitenteile (14, 14'). Diese Seitenteile, die mit dem Unterteil (11) verbunden und in eine den U-förmigen Kanal (13) erweiternde Lage abspreizbar sind, bilden zusammen mit dem Unterteil ein die Längsausnehmung (12) und den U-förmigen Kanal (13) umschließendes nachgiebiges Polster. Bei bestimmungsgemäßer Verwendung der Vorrichtung erstrecken sich die Schienenteile (11, 14, 14') beidseitig über eine Fraktur der dann in dem U-förmigen Kanal (13) aufgenommenen Extremität eines Patienten hinaus und sind mittels äußerer Umschlingungsmittel zwecks Fixation der Fraktur festgelegt.

Fig. 1

EP 0 332 960 A1

## Vorrichtung zum Schienen von Extremitäten, insbesondere von Unterschenkelfrakturen

Die Erfindung betrifft eine Vorrichtung zum Schienen von Extremitäten, insbesondere von Unterschenkelfrakturen, mit mehreren langgestreckten, bei bestimmungsgemäßer Verwendung sich beidseitig über eine Fraktur hinaus erstreckend anlegbaren und mittels äußerer Umschließungsmittel zwecks Fixation der Fraktur festlegbaren Schienenteilen.

Eine bekannte Vorrichtung dieser Art und Zweckbestimmung, deren druckschriftliche Vorbeschreibung allerdings nicht belegt werden kann, besteht aus mehreren langgestreckten und durch längslaufende Verstärkungen aus Holzleisten versteiften Schlauchschienen, die im Anwendungsfalle einzeln aufgeblasen und jeweils längslaufend in Abständen voneinander um eine zu schienende Extremität, etwa einen menschlichen Unterschenkel mit Fraktur, herumgelegt und dann mit Hilfe von beispielsweise Dreieckstüchern oder anderer Umschlingungsmittel festgebunden werden müssen.

Die bekannte Vorrichtung ist unbefriedigend insofern, als damit nur eine unzulängliche Fixation einer Fraktur gelingt und es dafür erheblicher Manipulationen bedarf.

Es ist auch schon eine sogenannte "Luftkammerschiene" bekannt, bei der es sich um einen kreisringförmigen Schlauch handelt, der in Längsrichtung in mehrere Kammern unterteilt ist. Bei bestimmungsgemäßer Verwendung muß dieser Schlauch auf die frakturbehaftete Extremität von derem distalen Ende aus aufgeschoben und dann aufgeblasen werden.

Die Handhabung auch dieser Vorrichtung zum Schienen von Extremitäten ist höchst unbefriedigend und für den betroffenen Patienten insbesondere bei Unterschenkelfrakturen sehr schmerzhaft, weil die manschettenartig ausgebildete Luftkammerschiene über den Fuß des Patienten aufgeschoben werden muß.

Bekannt sind aber auch die sogenannten "Drahtleiterschienen", bei denen es sich um langgestreckte Drahtleitern mit Quersprossen handelt, die bei Gebrauch mit Zellstoffbinden oder dergleichen umwickelt werden müssen, um eine gewisse Abpolsterung zu gewährleisten. Bei bestimmungsgemäßer Verwendung werden dann jeweils drei oder vier dieser Leitern längs einer frakturbehafteten Extremität angelegt und mittels Gurten oder ähnlicher Mittel befestigt. Unbefriedigend auch hierbei ist die erhebliche Manipulation bis zum Erreichen einer zu dem noch unzulänglichen Fixation einer Fraktur.

Die der Erfindung zugrundeliegende Aufgabe besteht demgegenüber in der Schaffung einer besonders einfach handhabbaren Vorrichtung zum Schienen von Extremitäten, die bei bestimmungsgemäßer Verwendung eine bessere Fixation einer Fraktur als die vorbekannten Vorrichtungen vermittelt und zu einer verringerten Patientenbelastung führt.

Gelöst ist diese Aufgabe dadurch, daß bei der Vorrichtung nach dem Oberbegriff des Schutzanspruchs 1 ein Unterteil eine einseitig offene, anatomisch geformte Längsausnehmung aufweist, daß sich an den Längskanten der Längsausnehmung zwei letztere zu einem U-förmigen Kanal ergänzende Seitenteil entlang erstrekken, die mit dem Unterteil verbunden und in eine den U-förmigen Kanal erweiternde Lage abspreizbar sind, und daß die Seitenteile und das Unterteil die Längsausnehmung und den U-förmigen Kanal umschließende nachgiebige Polster bilden.

Charakteristisch für die erfindungsgemäße Vorrichtung ist ein einseitig offener Kanal, der von nachgiebigen Polstern umgeben ist und infolge des Abspreizens der mit dem Unterteil verbundenen Seitenteile erweitert werden kann. Letzteres ermöglicht eine problemlose Handhabung insofern, als bei abgespreizten Seitenteilen eine frakturbehaftete Extremität eines Patienten, etwa eine Unterschenkelfraktur, in einfacher Weise in dem erweiterten Kanal gebettet und - nach einer in aller Regel erforderlichen Streckung - durch Gurte oder sonstige geeignete Umschließungsmittel die Schienenteile mit ihren den Kanal umgebenden Polstern hinreichend fest an die Extremität angedrückt und festgelegt werden können. Dadurch gelingt eine präzise Fixation der Fraktur ohne die Gefahr höchst unerwünschter und schädlicher Kompressionen.

Insbesondere ist letzteres der Fall, wenn gemäß einer Ausgestaltung der Erfindung die Seitenteile und das Unterteil als Formteile aus Schaumstoff, Schaumgummi oder einem ähnlichen nachgiebigen Material ausgebildet und durch außenseitig an diesen längsverlaufend angeordnete formstabile Schienen versteift sind. Bei diesen Schienen kann es sich mit Vorteil um an den Seitenteilen und am Unterteil außenseitig längsverlaufend angeordnete, insbesondere angeklebte Holzleisten handeln.

Angesichts der ausgezeichneten Strahlengängigkeit von Schaumstoff, Schaumgummi oder ähnlichem Material kann bei einer derartigen Ausbildung der Vorrichtung eine geschiente Extremität röntgenologisch untersucht und bis unmittelbar vor ihrer Versorgung im geschienten Zustand belassen werden. Im Vergleich zu bekannten Vorrichtungen kann dadurch die Belastung eines Patienten außerordentlich reduziert werden, zumal auch angesichts der abspreizbaren Seitenteile das Ausschienen ohne umständliche und für den Patienten schmerzhafte Manipulationen gelingt.

Bei der Ausbildung der Vorrichtung zum Schienen von Unterschenkelfrakturen sieht eine Weiterbildung der vorgenannten Ausgestaltung vor, daß der U-förmige Kanal an einem Stirnende von einem fest mit dem Unterteil verbundenen Fußteil abgeschlossen ist, das über die Längsausnehmung im Unterteil hinausragt, und daß die Seitenteile jeweils in ihren entsprechenden Stirnenden mit dem Fußteil fest verbunden, etwa verklebt, im übrigen jedoch über zumindest einen wesentlichen Teil ihrer Längserstreckungen aus ihren den U-förmigen Kanal seitlich begrenzenden Lagen abspreizbar sind.

Bei einer derartigen Ausbildung der Vorrichtung gelingt es, den U-förmigen Kanal infolge Abspreizens der Seitenteile vom distalen Ende der Vorrichtung aus trapezförmig zu erweitern. Dies hat sich für eine einfache Handhabung und weithin schmerzfreie Bettung einer Unterschenkelfraktur als vorteilhaft erwiesen.

Ein wesentlicher Mangel bekannter Vorrichtungen zum Schienen von Extremitäten besteht darin, daß es beim Transport eines Patienten mittels Krankenwagen zu Torsionen im Bereich einer geschienten Fraktur kommen kann, weil bei Transportfahrten Rollbewegungen des Patienten um seine Längsachse schlechterdings unvermeidbar sind, die sich dann bis zu der Fraktur fortsetzen können.

Hier schafft eine Weiterbildung der Erfindung dergestalt Abhilfe, daß die der anatomisch geformten Längsausnehmung gegenüberliegende Unterseite des Unterteils - im Querschnitt gesehen - konvex ausgebildet ist, und/oder daß die Längskanten zwischen den Außenseiten und der Unterseite des Unterteils abgerundet sind. Eine so ausgelegte Vorrichtung vermag den begrenzten Rollbewegungen eines Patienten beim Transport im wesentlichen ungehindert zu folgen, so daß die Gefahr von Torsionen im Bereich einer Fraktur weitestgehend ausgeschaltet ist.

Bei der Ausbildung der Vorrichtung zum Schienen von Unterschenkelfrakturen ist eine besonders günstige Bettung und Fixation auch erreichbar, wenn gemäß einer anderen Weiterbildung der vom Unterteil und den Seitenteilen umschlossene U-förmige Kanal mit einer distalen Unterschenkel-Fußeinlage in Form einer eingelegten Matte aus Schaumstoff, Schaumgummi oder einem ähnlichen Material versehen ist, die sich der Form eines zu bettenden Unterschenkels gut anpaßt.

Gemäß einer nochmaligen Weiterbildung kann es sich im Interesse einer einfachen Handhabung und vor allem einer guten Anpaßbarkeit an die individuellen Bedürfnissen des jeweiligen Patienten bei den äußeren Umschlingungsmitteln zum Festlegen der Schienenteile zwecks Fixation einer Fraktur um in Schienenlängsrichtung in vorbestimmten Abständen voneinander angeordnete Gurte mit Klettbandverschlüssen handeln, die eine schnelle und sichere Festlegung der Schienenteile zueinander, vor allem aber eine stufenlose Einstellbarkeit der Vorrichtung ermöglichen.

Schließlich hat es sich unter Hygienegesichtspunkten auch als vorteilhaft erwiesen, wenn das Unterteil und die Seitenteile von einem auch den U-förmigen Kanal auskleidenden Kunststoffbeutel umhüllt sind, der als Wegwerfartikel nach jedem bestimmungsgemäßen Gebrauch durch einen neuen Beutel ersetzt werden kann.

Anhand der beigefügten Zeichnung soll nachstehend eine Ausführungsform der erfindungsgemäßen Vorrichtung erläutert werden. In schematischen Ansichten zeigen:

Fig. 1 eine Vorrichtung zum Schienen von Unterschenkelfrakturen mit mittels dreier Gurtungen in bestimmungsgemäßer Schließlage gehaltenen Seitenteilen in einer perspektivischen Gesamtansicht,

Fig. 2 eine stirnseitige Ansicht der geschlossenen Vorrichtung gemäß Pfeil II in Fig. 1 mit Blick zum distalen Ende, jedoch bei weggelassenen Gurtungen,

Fig. 3 eine Ansicht der Vorrichtung ähnlich Fig. 1 jedoch bei offenen Gurtungen und seitlich leicht abgespreizten Seitenteilen.

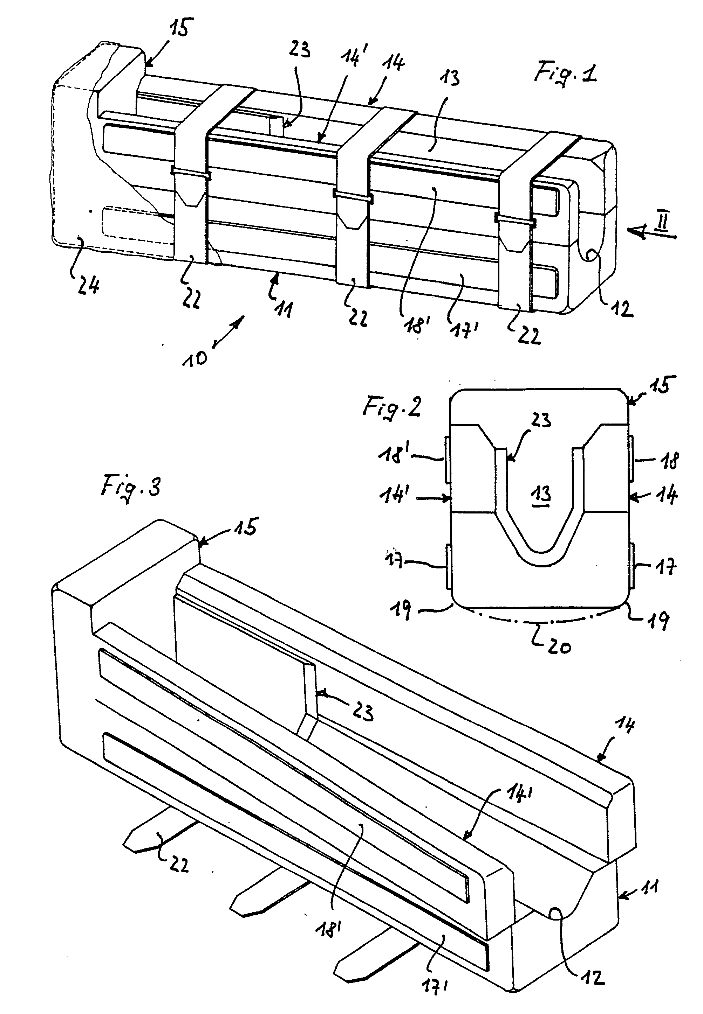

Insbesondere die Fig. 1 und 3 zeigen in anschaulicher Weise den Aufbau der Vorrichtung 10 zum Schienen von Unterschenkelfrakturen aus einem langgestreckten Unterteil 11 mit einer Längsausnehmung 12, zwei die Längsausnehmung zu einem U-förmigen Kanal 13 ergänzenden Seitenteilen 14, 14' und einem Fußteil 15. Bei dem Unterteil 11, den Seitenteilen 14, 14' und dem Fußteil 15' handelt es sich um Formteile aus Schaumstoff, Schaumgummi oder einem ähnlichen nachgiebigen Material. Das Fußteil 15 ist mit dem Unterteil 11 an dessen distalen Stirnende fest verbunden, etwa verklebt, und ragt über letzteres auf Seiten der Längsausnehmung 12 hinaus. Die Seitenteile 14, 14' sind in einem dem Breitenmaß des U-förmigen Kanals 13 entsprechenden Abstand voneinander ebenfalls mit ihren distalen Stirnenden fest mit dem Fußteil 1 5 verbunden, z.B. verklebt, und dadurch über praktisch die gesamte Längenerstreckung der Vorrichtung 10 aus ihren in Fig. 1 gezeigten bestimmungsgemäßen Schließlagen in die aus Fig. 3 ersichtlichen Öffnungslagen abspreizbar.

Zur Längsversteifung des Unterteils 11 und der beiden Seitenteile 14, 14', die aus elastisch nachgiebigem Material bestehen und daher von Hause aus nur begrenzte Formstabilität aufweisen, sind mit dem Unterteil an dessen beiden Außenseiten und ebenfalls außenseitig mit jedem Seitenteil längs verlaufende Versteifungsschienen 17, 17′ bzw. 18, 18′ in Form stabiler Holzleisten schubfest verbunden, und zwar vorzugsweise mit den genannten Teilen verklebt. Schließlich sind die sich zwischen der Unterseite des Unterteils und dessen Außenseiten erstreckenden Längskanten 19, 19′ abgerundet und im Bedarfsfalle kann die Unterseite auch konvex ausgebildet sein, wie dies in Fig. 3 bei 20 strichpunktiert angedeutet ist.

Bei bestimmungsgemäßer Verwendung der erfindungsgemäßen Vorrichtung 10 ist ein mit einer Fraktur behafteter Unterschenkel eines Patienten in dem U-förmigen Kanal 13 aufgenommen und durch die insbesondere aus Fig. 1 in ihren Schließlagen ersichtlichen Gurtungen 22 fixiert. Beim Anlegen der Vorrichtung befinden sich die Seitenteile 14, 14′ in ihren abgespreizten Lagen, wie dies in Fig. 3 angedeutet ist. Der durch das Abspreizen der Seitenteile vom distalen Ende aus sich erweiternde Kanal ermöglicht eine einfache Bettung eines Unterschenkels, wobei die am distalen Ende in den U-förmigen Kanal eingelegte Unterschenkel-Fußeinlage 23 eine gute Abstützung des Knöchelbereichs gewährleistet. Nach der Bettung eines Unterschenkels in der Längsausnehmung 12 des Unterteils werden die Seitenteile 14, 14′ aus ihren Abspreizlagen zurückgeführt und an den gebetteten Unterschenkel des Patienten angelegt sowie durch die insbesondere aus Fig. 1 ersichtlichen Gurtungen 22, die mit Klettbandverschlüssen versehen sind, in solcher Weise festgelegt, daß eine eindeutige Fixation der Unterschenkelfraktur gewährleistet ist. Angesichts der Ausbildung des Unterteils und der Seitenteile als Formteile aus Schaumstoff, Schaumgummi oder einem ähnlichen nachgiebigen Material ist der gebettete Unterschenkel von weichen Polstern umgeben, so daß unbeschadet einer präzisen Fixation der Fraktur die Gefahr von Kompressionen weitestgehend vermieden ist.

Ein besonderer Vorteil der erfindungsgemäßen Vorrichtung besteht darin, daß beispielsweise bei der Aufnahme eines Patienten in einer Unfall-Ambulanz eine geschiente Extremität vom Aufnahmearzt in Augenschein genommen werden kann, ohne daß schmerzhafte Manipulationen vorgenommen werden müssen. Zu diesem Zwecke müssen lediglich die Gurtungen gelöst und die Seitenteile 14, 14′ abgespreizt werden. Die Extremität bleibt dabei stabil auf dem anatomisch geformten Unterteil 11 liegen. Nach einer derartigen Inaugenscheinnahme oder auch Untersuchung kann die Vorrichtung wieder geschlossen und der Patient zum Ort seiner Behandlung weiter transportiert werden.

In Fig. 1 ist im Bereich des distalen Endes der Vorrichtung ein im übrigen weggebrochen gezeichneter Einweg-Kunststoffbeutel 24 dargestellt, der das Unterteil 11 und die Seitenteile 14, 14′ vollständig umschließt und auch den U-förmigen Kanal 13 mit der Längsausnehmung 12 im Unterteil 11 auskleidet. Dieser Kunststoffbeutel, der nach jeder bestimmungsgemäßen Verwendung ersetzbar ist, gewährleistet einen hygienischen Gebrauch der Vorrichtung.

## Ansprüche

1. Vorrichtung zum Schienen von Extremitäten, insbesondere von Unterschenkelfrakturen, mit mehreren langgestreckten, bei bestimmungsgemäßer Verwendung sich beidseitig über eine Fraktur hinauserstreckend anlegbaren und mittels äußerer Umschlingungsmittel zwecks Fixation der Fraktur festlegbaren Schienenteilen, dadurch gekennzeichnet,
daß ein Unterteil (11) eine einseitig offene, anatomisch geformte Längsausnehmung (12) aufweist, daß sich an den Längskanten der Längsausnehmung zwei letztere zu einem U-förmigen Kanal (13) ergänzende Seitenteile (14, 14′) entlang erstrecken, die mit dem Unterteil verbunden und in eine den U-förmigen Kanal erweiternde Lage abspreizbar sind, und daß die Seitenteile und das Unterteil die Längsausnehmung und den U-förmigen Kanal umschließende nachgiebige Polster bilden.

2. Vorrichtung nach Anspruch 1, dadurch gekennzeichnet, daß die Seitenteile (14, 14′) und das Unterteil (11) als Formteile aus Schaumstoff, Schaumgummi oder einem ähnlichen nachgiebigen Material ausgebildet und durch außenseitig an diese längsverlaufend angeordnete formstabile Schienen (17, 17′; 18, 18′) versteift sind.

3. Vorrichtung nach Anspruch 2, dadurch gekennzeichnet, daß es sich bei den zur Längsversteifung an den Seitenteilen (14, 14′) und am Unterteil (11) außenseitig längsverlaufend angeordneten formstabilen Schienen um insbesondere angeklebte Holzleisten (17, 17′; 18, 18′) handelt.

4. Vorrichtung nach Anspruch 2 oder 3, dadurch gekennzeichnet, daß bei der Ausbildung zum Schienen von Unterschenkelfrakturen der U-förmige Kanal (13) an einem Stirnende von einem fest mit dem Unterteil (11) verbundenen Fußteil (15) abgeschlossen ist, das über die Längsausnehmung (12) hinausragt, und daß die Seitenteile (14, 14′) jeweils mit ihren entsprechenden Stirnenden mit dem Fußteil fest verbunden, etwa verklebt, im übrigen jedoch über zumindest einen wesentlichen Teil ihrer Längserstreckungen aus ihren den U-förmigen Kanal seitlich begrenzenden Lagen abspreizbar sind.

5. Vorrichtung nach einem der Ansprüche 2 bis 4, dadurch gekennzeichnet, daß die der anatomisch geformten Längsausnehmung (12) gegenüberliegende Unterseite des Unterteils - im Querschnitt gesehen - konvex ausgebildet ist.

6. Vorrichtung nach einem der Ansprüche 2 bis 5, dadurch gekennzeichnet, daß die Längskanten (19, 19') zwischen den Außenseiten und der Unterseite des Unterteils (11) abgerundet sind.

7. Vorrichtung nach einem der Ansprüche 1 bis 6, dadurch gekennzeichnet, daß bei der Ausbildung zum Schienen von Unterschenkelfrakturen der vom Unterteil (11) und den Seitenteilen (14, 14') umschlossene U-förmige Kanal (13) mit einer distalen Unterschenkel-Fußeinlage (23) in Form einer eingelegten Matte aus Schaumstoff, Schaumgummi oder einem ähnlichen Material versehen ist.

8. Vorrichtung nach einem der Ansprüche 1 bis 7, dadurch gekennzeichnet, daß es sich bei den äußeren Umschlingungsmitteln zum Festlegen der Schienenteile (11, 14, 14') zwecks Fixation einer Fraktur um in Schienenlängsrichtung in vorbestimmten Abständen voneinander angeordnete Gurte (22) mit Klettbandverschlüssen handelt.

9. Vorrichtung nach einem der Ansprüche 1 bis 8, gekennzeichnet durch einen das Unterteil (11) und die Seitenteile (14, 14') umhüllenden und auch den U-förmigen Kanal (13) auskleidenden Kunststoffbeutel (24).

*Fig.1*

*Fig.2*

*Fig.3*

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-2 984 239 (TAYLER)<br>* Insgesamt *<br>--- | 1-5 | A 61 F 5/04 |
| Y | CH-A- 447 474 (KEEL)<br>* Spalte 1, Zeile 34 - Spalte 3, Zeile 10; Figuren *<br>--- | 1-5 | |
| A | FR-A-1 325 526 (THOMAS)<br>* Figuren 1,3 *<br>--- | 6 | |
| A | US-A-4 505 269 (DAVIES et al.)<br>* Spalte 4, Zeilen 27-34; Spalte 6, Zeilen 15-31 *<br>--- | 7,8 | |
| A | US-A-3 992 856 (McGOVERN)<br>* Anspruch 1; Figuren *<br>----- | 9 | |

RECHERCHIERTE SACHGEBIETE (Int. Cl.4)

A 61 F
A 61 B

Der vorliegende Recherchenbericht wurde für alle Patentansprüche erstellt

| Recherchenort | Abschlußdatum der Recherche | Prüfer |
|---|---|---|
| DEN HAAG | 14-06-1989 | STEENBAKKER J. |